# EUROPÄISCHE PATENTSCHRIFT

(11) **EP 1 144 407 B1**
(45) Veröffentlichungstag und Bekanntmachung des Hinweises auf die Patenterteilung: **04.06.2003**
(21) Anmeldenummer: 00904877.8
(22) Anmeldetag: 05.01.2000
(51) Int. Cl.: C07D 417/06, C07D 413/06, C07D 401/06, A01N 43/88, A01N 43/66, A01N 43/78

(54) **ZYKLISCHE GUANIDIN-DERIVATE UND IHRE VERWENDUNG ALS PESTIZIDE**
CYCLIC GUANIDINE DERIVATIVES AND THEIR USE AS PESTICIDES
DERIVES DE GUANIDINE CYCLIQUES ET LEUR UTILISATION COMME PESTICIDES

(30) Priorität: 08.01.1999 DE 19900519
(43) Veröffentlichungstag der Anmeldung: 17.10.2001
(73) Patentinhaber: Bayer CropScience AG, 40789 Monheim (DE)
(72) Erfinder: WAGNER, Klaus, D-83115 Neubeuern (DE); ERDELEN, Christoph, D-42799 Leichlingen (DE); TURBERG, Andreas, D-42781 Haan (DE); MENCKE, Norbert, D-51381 Leverkusen (DE); HANSEN, Olaf, D-40789 Monheim (DE)
(86) Internationale Anmeldenummer: EP0000044
(87) Internationale Veröffentlichungsnummer: WO00040582

(56) Entgegenhaltungen:
- EP-A- 0 483 055
- EP-A- 0 580 553
- WO-A-91/01978
- WO-A-98/06710

## Beschreibung

Die vorliegende Anmeldung betrifft neue Guanidin-Derivate, ein Verfahren zu ihrer Herstellung und ihre Verwendung zur Bekämpfung von tierischen Schädlingen.

Es ist bereits bekannt, dass bestimmte heterocyclische Verbindungen insektizide Eigenschaflen aufweisen (vgl. z.B. WO-A-910 1978, WO-A-98 067 10, EP-A 0 386 565, EP-A 0 428 941, EP-A 0 483 055, EP-A 0 580 553, US 5 032 589 und US 5 034 524).

Die Wirksamkeit bzw. Wirkungsbreite dieser Verbindungen ist jedoch insbesondere bei niedrigen Aufwandmengen und Konzentrationen nicht immer in allen Anwendungsgebieten ganz zufriedenstellend.

Es wurden neue Guanidin-Derivate der Formel (I) gerunden, 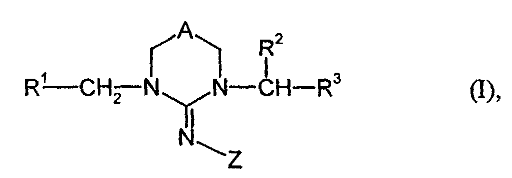 in welcher
- R¹: für einen fünf- oder sechsgliedrigen Heterocyclus steht, welcher 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt - und welcher gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenalkenyloxy, Alkinyloxy, Alkylthio, Halogenalkylthio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Arylthio, Arylamino, Aralkyl, Formylamino, Alkylcarbonylamino, Formyl, Carbamoyl, Alkylcarbonyl und/oder Alkoxycarbonyl substituiert ist,
- R²: für Wasserstoff oder Alkyl steht,
- R³: für einen Rest aus der Reihe -OR⁴, -OCOR⁵, -OCOOR⁶, -OCONR⁷R⁸, -OSO₂R⁹ und -S(O)ₙR¹⁰ steht, wobei
R⁴_{,} R⁵_{,} R⁶ und R¹⁰ unabhängig voneinander für einen Rest aus der Reihe Alkyl, Alkoxyalkyl, Halogenalkyl, Alkenyl, Alkinyl, Alkylaminoalkyl, Dialkylaminoalkyl, gegebenenfalls substituiertes Cycloalkyl und jeweils gegebenenfalls substituiertes Phenyl und Benzyl stehen,
n für 0, 1 oder 2 steht,
R⁷ und R⁸ unabhängig voneinander für einen Rest aus der Reihe Wasserstoff, Alkyl, Alkenyl und jeweils gegebenenfalls substituiertes Phenyl und Benzyl stehen und
R⁹ für Alkyl oder gegebenenfalls substituiertes Phenyl steht,
- A: für Sauerstoff, Schwefel oder für NR¹¹ steht, wobei
R¹¹ für einen Rest aus der Reihe Alkyl, Alkenyl, Alkinyl, Alkoxy, gegebenenfalls substituiertes Cycloalkyl und gegebenenfalls substituiertes Aralkyl steht und
- Z: für Cyano oder Nitro steht.

Weiterhin wurde gefunden, dass man die Verbindungen der Formel (I) erhält, wenn man Verbindungen der Formel (II) 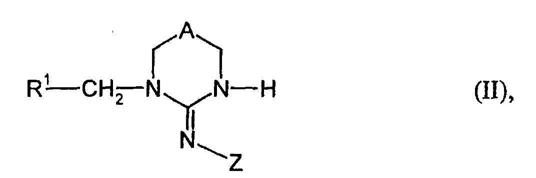 in welcher
R¹, A und Z die oben angegebenen Bedeutungen haben,
mit Halogenverbindungen der Formel (III) 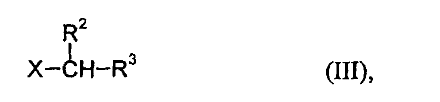 in welcher
R² und R³ die oben angegebenen Bedeutungen haben und
- X: für Halogen (insbesondere Chlor oder Brom) steht,
in Gegenwart einer Base und gegebenenfalls in Gegenwart eines Verdünnungsmittels umsetzt.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- R¹: steht bevorzugt für einen fünf- oder sechsgliedrigen Heterocyclus aus der Reihe Pyrazolyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl, welcher gegebenenfalls durch einen oder zwei, bevorzugt einen Substituenten aus der Reihe Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist), oder C₁-C₂-Alkylsulfonyl (welches gegenenenfalls durch Fluor und/oder Chlor substituiert ist) substituiert ist.
- R²: steht bevorzugt für Wasserstoff oder C₁-C₆-Alkyl.
- R³: steht bevorzugt für einen Rest aus der Reihe -OR⁴, -OCOR⁵, -OCOOR⁶, -OCONR⁷R⁸, -OSO₂R⁹ und -S(O)ₙR¹⁰.
R⁴, R⁵, R⁶ und R¹⁰ stehen unabhängig voneinander bevorzugt für einen Rest aus der Reihe C₁-C₁₂-Alkyl, C₁-C₄-Alkoxy-C₁-C₄-alkyl, C₁-C₄-Halogenalkyl mit 1 bis 5 gleichen oder verschiedenen Halogenatomen, wie Fluor-, Chlor- und Bromatomen; C₂-C₄-Alkenyl, C₂-C₄-Alkinyl, C₁-C₄-Alkylamino-C₁-C₄-alkyl, Di(C₁-C₄)-alkylamino-C₁-C₄-alkyl, für gegebenenfalls einfach bis dreifach, gleich oder verschieden substituiertes C₃-C₆-Cycloalkyl, wobei als Substituenten vorzugsweise Halogen, C₁-C₄-Alkyl und C₁-C₄-Halogenalkyl mit 1 bis 5 gleichen oder verschiedenen Halogenatomen, wie F-, C1- und Br-Atomen in Frage kommen, oder für jeweils gegebenenfalls einfach bis dreifach, gleich oder verschieden substituiertes Phenyl oder Benzyl, wobei jeweils als Substituenten am Phenylring vorzugsweise Halogen, C₁-C₄-Alkyl, C₁-C₄-Alkoxy, C₁-C₄-Halogenalkyl und C₁-C₄-Halogenalkoxy mit jeweils 1 bis 5 gleichen oder verschiedenen Halogenatomen, wie Fluor-, Chlor- und Bromatomen und Nitro in Frage kommen.
n steht bevorzugt für 0, 1 oder 2.
R⁷ und R⁸ stehen unabhängig voneinander bevorzugt für einen Rest aus der Reihe Wasserstoff, C₁-C₆-Alkyl, C₂-C₄-Alkenyl und für jeweils gegebenenfalls einfach bis dreifach, gleich oder verschieden substituiertes Phenyl oder Benzyl, wobei jeweils als Substituenten am Phenylring vorzugsweise Halogen, C₁-C₄-Alkyl, C₁-C₄-Alkoxy, C₁-C₄-Halogenalkyl und C₁-C₄-Halogenalkoxy mit jeweils 1 bis 5 gleichen oder verschiedenen Halogenatomen, wie Fluor-, Chlor- und Bromatomen in Frage kommen.
R⁹ steht bevorzugt für C₁-C₄-Alkyl oder für gegebenenfalls einfach bis dreifach, gleich oder verschieden substituiertes Phenyl, wobei als Substituenten Halogen, C₁-C₄-Alkyl, C₁-C₄-Alkoxy und C₁-C₄-Halogenalkyl und C₁-C₄-Halogenalkoxy mit jeweils 1 bis 5 gleichen oder verschiedenen Halogenatomen, wie Fluor-, Chlor- und Bromatomen in Frage kommen.
- A: steht bevorzugt für Sauerstoff, Schwefel oder für -NR¹¹.
R¹¹ steht bevorzugt für C₁-C₄-Alkyl, C₂-C₄-Alkenyl, C₂-C₄-Alkinyl, C₁-C₄-Alkoxy, für gegebenenfalls einfach bis dreifach, gleich oder verschieden substituiertes C₅-C₆-Cycloalkyl, wobei als Substituenten bevorzugt Halogen, C₁-C₄-Alkyl, C₁-C₄-Alkoxy, C₁-C₄-Halogenalkyl und C₁-C₄-Halogenalkoxy mit jeweils 1 bis 5 gleichen oder verschiedenen Halogenatomen, wie Fluor-, Chlor- und Bromatomen in Frage kommen, oder für einfach bis dreifach, gleich oder verschieden substituiertes Phenyl-C₁-C₄-alkyl, wobei als Substituenten bevorzugt Halogen, C₁-C₄-Alkyl, C₁-C₄-Alkoxy und C₁-C₄-Halogenalkyl und C₁-C₄-Halogenalkoxy mit jeweils 1 bis 5 gleichen oder verschiedenen Halogenatomen, wie Fluor-, Chlor- und Bromatomen in Frage kommen.
- Z: steht bevorzugt für Cyano oder Nitro.
- R¹: steht besonders bevorzugt für 6-Chlor-3-pyridyl (6-Chlor-pyridin-3-yl) oder für 2-Chlor-5-thiazolyl (2-Chlor-thiazol-5-yl).
- R²: steht besonders bevorzugt für Wasserstoff oder C₁-C₅-Alkyl.
- R³: steht besonders bevorzugt für einen Rest aus der Reihe -OR⁴, -OCOR⁵, -OCOOR⁶, -OCONR⁷R⁸, -OSO₂R⁹ und -S(O)ₙR¹⁰.
R⁴_{,} R⁵, R⁶ und R¹⁰ stehen unabhängig voneinander besonders bevorzugt für C₁-C₈-Alkyl, C₁-C₂-Alkoxy-C₁-C₂-alkyl, C₁-C₂-Halogenalkyl mit 1 oder 2 gleichen oder verschiedenen Halogenatomen, wie Fluor-, Chlor- und Bromatomen; Allyl, Propargyl, C₁-C₂-Alkylamino-C₁-C₂-alkyl, Di(C₁-C₂)-alkylamino-C₁-C₂-alkyl, Cyclopropyl, Cyclopentyl, Cyclohexyl oder für jeweils gegebenenfalls einfach oder zweifach, gleich oder verschieden substituiertes Phenyl oder Benzyl, wobei jeweils als Substituenten am Phenylring Fluor, Chlor, Brom, Methyl, Methoxy, Trifluormethyl, Trifluormethoxy und Nitro in Frage kommen.
n steht besonders bevorzugt für 0, 1 oder 2.
R⁷ und R⁸ stehen unabhängig voneinander besonders bevorzugt für einen Rest aus der Reihe Wasserstoff, C₁-C₄-Alkyl, Vinyl, Allyl oder für jeweils gegebenenfalls einfach oder zweifach, gleich oder verschieden substituiertes Phenyl oder Benzyl, wobei jeweils als Substituenten am Phenylring Fluor, Chlor, Brom, Methyl, Methoxy, Trifluormethyl und Trifluormethoxy in Frage kommen.
R⁹ steht besonders bevorzugt für Methyl, Ethyl oder gegebenenfalls einfach oder zweifach, gleich oder verschieden substituiertes Phenyl, wobei als Substituenten Fluor, Chlor, Brom, Methyl, Methoxy, Trifluormethyl und Trifluormethoxy in Frage kommen.
- A: steht besonders bevorzugt für Sauerstoff, NCH₃, NCH₂CH=CH₂; 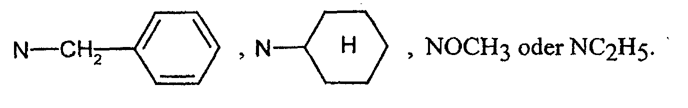
- Z: steht besonders bevorzugt für Cyano oder Nitro.

Bevorzugte erfindungsgemäße Verbindungen sind Stoffe der Formeln (I A) bis (I D): 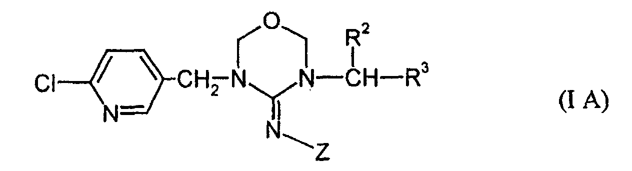 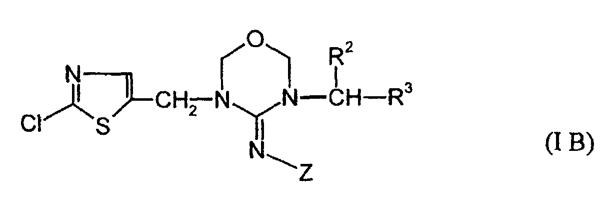 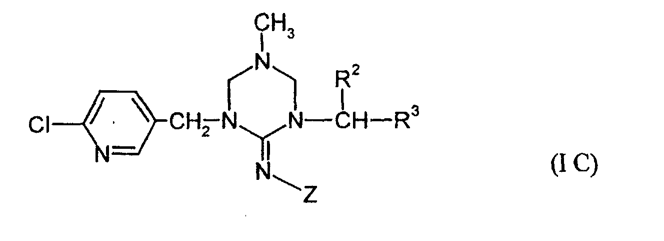 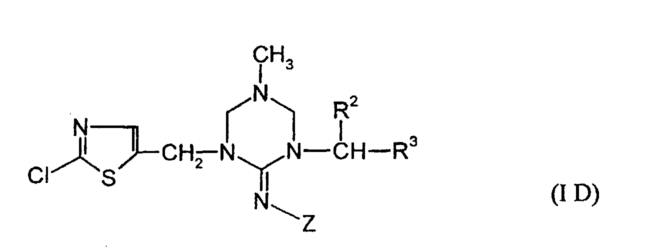 in welchen
- R², R³ und Z: für die obengenannten allgemeinen, bevorzugten und besonders bevorzugten Bedeutungen stehen.

Bevorzugte erfindungsgemäße Verbindungen sind auch Stoffe der Formeln (I A-1), (I A-2), (I B-1), (I B-2), (I C-1), (I C-2), (I D-1) und (I D-2): 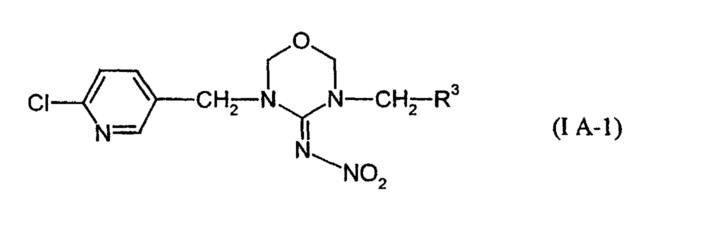 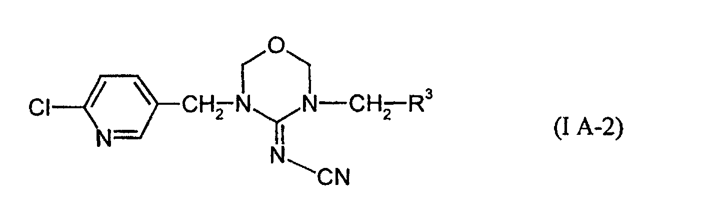 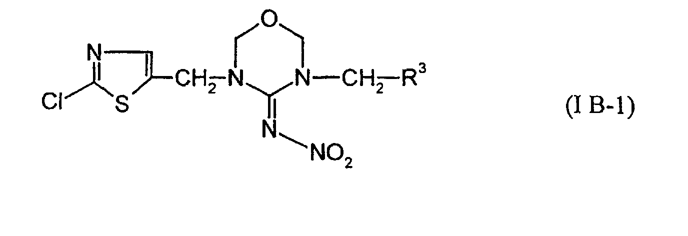 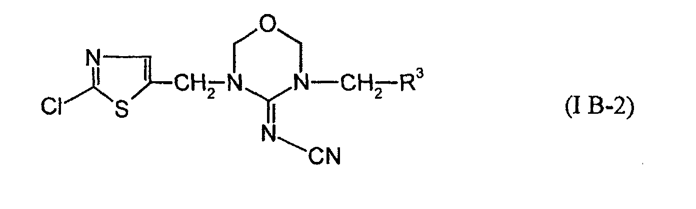 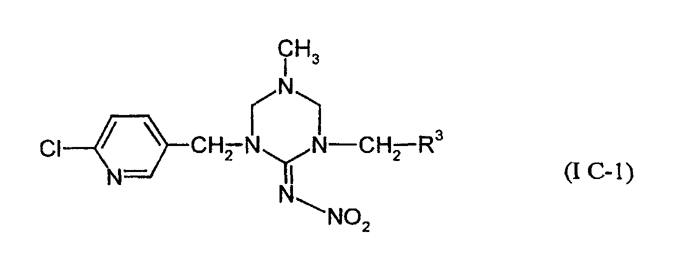 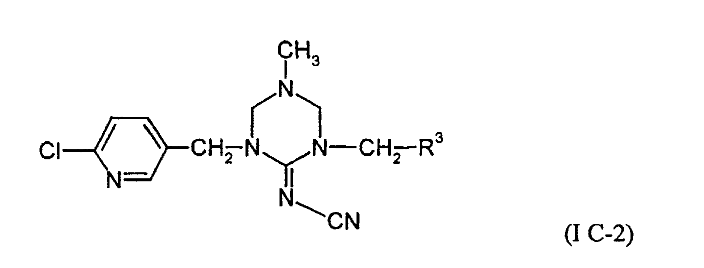 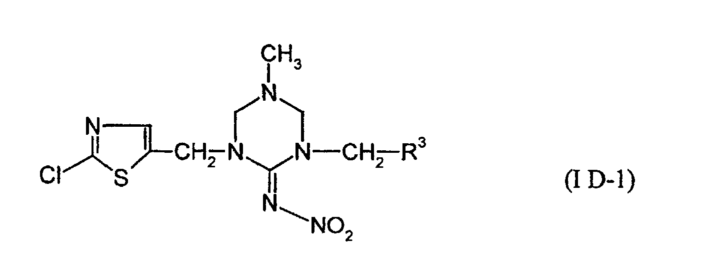 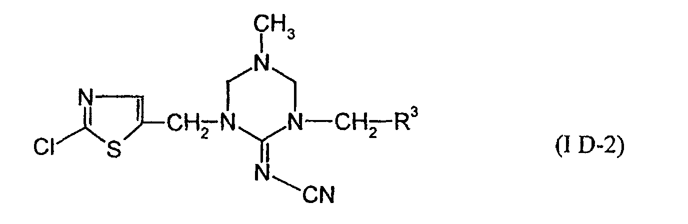 in welchen
- R³: für die oben genannten allgemeinen, bevorzugten und besonders bevorzugten Bedeutungen steht.

Die oben aufgeführten allgemeinen oder in Vorzugsbereichen aufgeführten Restedefinitionen bzw. Erläuterungen gelten für die Endprodukte und für die Ausgangsund Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

In den oben und nachstehend aufgeführten Restedefinitionen sind Kohlenwasserstoffreste, wie Alkyl - auch in Verbindung mit Heteroatomen wie Alkoxy - soweit möglich jeweils geradkettig oder verzweigt.

In den oben und nachstehend aufgeführten Restedefinitionen steht, sofern nichts anderes angegeben ist, Halogen(atome) für F, Cl, Br und Iod(atome), bevorzugt für F, Cl und Br(atome), besonders bevorzugt für F und Cl(atome).

Im einzelnen seien außer den Herstellungsbeispielen die folgenden Verbindungen genannt:

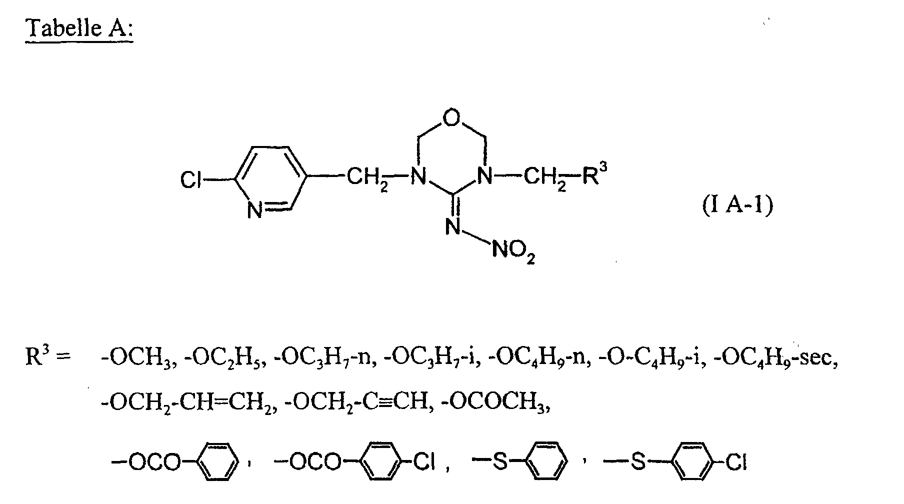

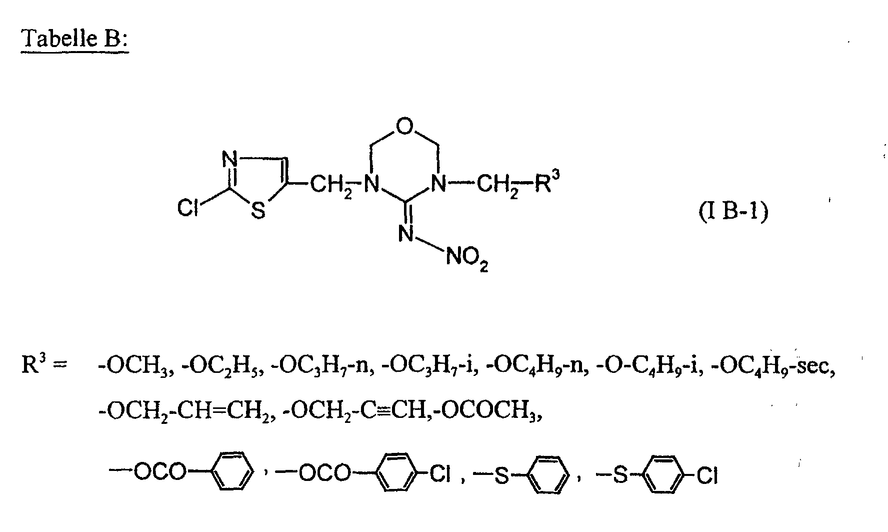

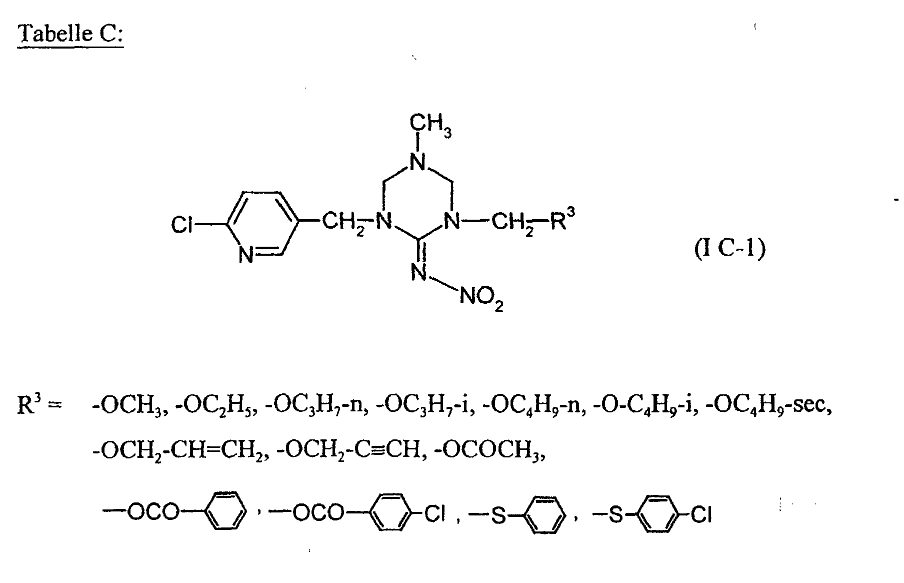

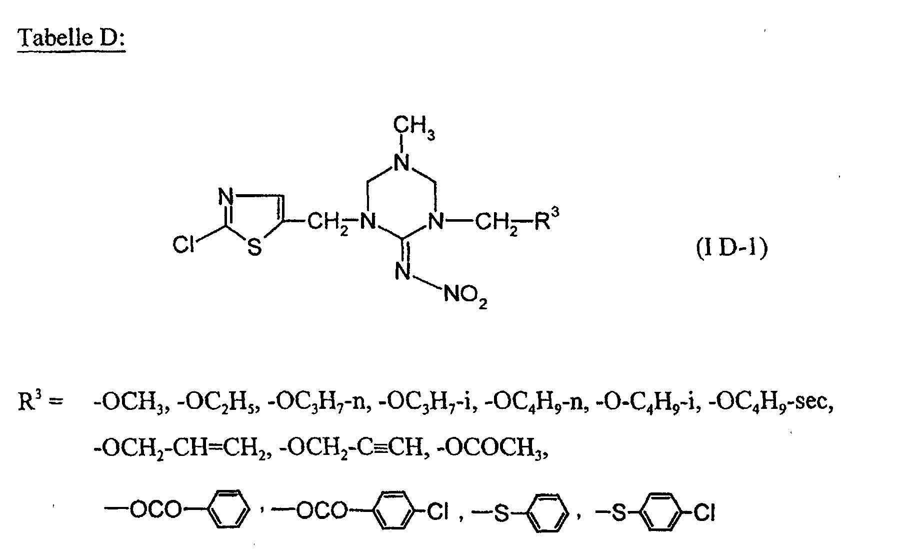

Verwendet man beispielsweise 3-(6-Chlor-pyridin-3-yl-methyl)-4-nitroimino-perhydro-1,3,5-oxadiazin und Propoxymethylchlorid als Ausgangsstoffe, so kann der Verlauf des erfindungsgemäßen Verfahrens durch das folgende Reaktionsschema wiedergegeben werden:

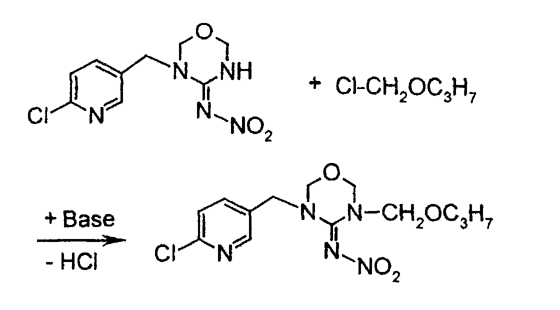

Die zur Durchführung des erfindungsgemäßen Verfahrens als Ausgangsstoffe benötigten Verbindungen der Formel (II) sind bekannt (vgl. z.B. EP-A 0 386 565, EP-A 0 428 941, EP-A 0 483 055, EP-A 0 580 553, US 5 032 589 und US 5 034 524) und/oder können auf die dort beschriebene Art und Weise erhalten werden.

Die weiterhin beim erfindungsgemäßen Verfahren als Ausgangsstoffe zu verwendenden Halogenverbindungen der Formel (III) sind allgemein bekannte Verbindungen der Organischen Chemie und/oder nach allgemein bekannten Methoden erhältlich.

Das erfindungsgemäße Verfahren zur Herstellung der neuen Verbindungen der Formel (I) wird vorzugsweise unter Verwendung von Verdünnungsmitteln durchgeführt: Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Das erfindungsgemäße Verfahren wird in Gegenwart einer Base durchgerührt. Als Basen können bei dem erfindungsgemäßen Verfahren alle üblichen Protonenakzeptoren eingesetzt werden. Vorzugsweise verwendbar sind Alkali- oder Erdalkalihydroxide, Alkali- oder Erdalkalihydride, Alkali- oder Erdalkalicarbonate oder -hydrogencarbonate oder Stickstoffbasen. Genannt seien beispielsweise Natriumhydroxid, Calciumhydroxid, Natriumhydrid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktionstemperaturen können bei dem erfindungsgemäßen Verfahren in einem größeren Bereich variiert werden. Im allgemeinen arbeitet man bei Temperaturen zwischen -40°C und +200°C, bevorzugt zwischen -10°C und 100°C, besonders bevorzugt zwischen 10°C und 50°C.

Das erfindungsgemäße Verfahren kann sowohl bei Normaldruck als auch unter erhöhtem Druck durchgeführt werden.

Das erfindungsgemäße Verfahren kann sowohl unter atmosphärischen Bedingungen als auch unter Schutzgasatmosphäre durchgeführt werden.

Bei der Durchführung des erfindungsgemäßen Verfahrens zur Herstellung der Verbindungen der Formel (I) setzt man im allgemeinen pro Mol Verbindungen der Formel (II) 1 bis 3 Mol, vorzugsweise 1 bis 2 Mol an Halogenverbindungen der Formel (III) ein.

Aufarbeitung und Isolierung der Endprodukte erfolgen in allgemein bekannter Art und Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (1) zeichnen sich insbesondere durch eine hohe insektizide Wirksamkeit aus.

Sie lassen sich mit besonders gutem Erfolg zur Bekämpfung pflanzenschädigender Blattinsekten einsetzen, wie beispielsweise gegen die Pfirsichblattlaus (Myzus persicae) sowie gegen die Larven des Meerrettich-Käfers (Phaedon cochleariae).

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Pflanzenteile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhiozome aufgeführt werden. Zu den Pflanzenteilen gehört auch vegetatives und generatives Vermehrungsmaterial, beispielsweise Stecklinge, Knollen, Rhizome, Ableger und Samen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylhamstoffe, durch Mikroorganismen hergestellte Stoffe u.a.

### Besonders günstige Mischpartner sind z.B. die folgenden:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazöl, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpidonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-ß-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-( 1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1 -(2-Methyl-1 -naphthalenyl)-1H-pyrrol-2,5-dion,
1 -(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1 - [[2-(4-Chlorphenyl)-3-phenyloxiranyl]-methyl]- 1H-1,2,4-triazol,
1-[ 1-[2-[(2,4-Dichlorphenyl)-methoxy]-phenyl]-ethenyl]-1H-imidazol,
1 Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3 -Triiod-2-propenyl)-2H-tetrazol,
2-[(1 -Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1 -[4-(difluormethoxy)-phenyl]-1H-pyrrol-2,5-dion,
3,5-Dichlor-N-[cyan[(1-methyl-2-propynyl)-oxy]-methyl]-benzamid,
3 -(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-( 1,1 -Dimethylethyl)-N-ethyl-N-propyl- 1 ,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl}-oxy]-2,5-thiophendicarboxylat,
cis-1 -(4-Chlorphenyl)-2-( 1 H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1 -Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectine, AZ 60541, Azadirachtin, Azinphos A, Azinphos M, Azocyclotin, AKD 1022,
Bacillus thuringiensis, Bendiocarb, Benfüracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofüran, Carbophenothion, Carbosulfan, Cartap, CGA 157 419, CGA 184 699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diaeloden, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb, TI 435, Thiamethoxam,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen die erfindungsgemäßen Verbindungen eine gute Wirkung gegen Fliegen (Musca domestica) sowie eine gute entwicklungshemmende Wirkung gegen Fliegenlarven von Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brurineus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus
Hautflügler wie
   Sirex juvencus, Urocerus gigas, Urocerus gigas taignus, Urocerus augur
Termiten wie
   Kalotermes flavicollis, Cryptotermes brevis, Heterotermes indicola, Reticulitermes flavipes, Reticulitermes santonensis, Reticulitermes lucifigus, Mastotermes darwiniensis, Zootermopsis nevadensis, Coptotermes formosanus.
Borstenschwänze, wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittels oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische, polar organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällung vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon. .

Als Lösungs- bzw. Verdünnungsmittel kommt insbesondere auch Wasser in Frage, gegebenenfalls in Mischung mit einem oder mehreren der obengenannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, lmidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

### Herstellungsbeispiele

### Beispiel 1

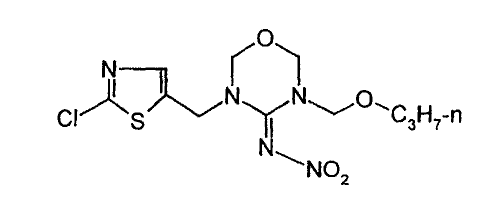

1,39 g (5 mMol) 3-(2-Chlor-thiazol-5-yl-methyl)4-nitroiminino-perhydro-1,3,5-oxadiazin in 15 ml absolutem Dimethylformamid werden unter Rühren portionsweise mit 0,24 g (6 mMol) Natriumhydrid versetzt. Nach 15-minütigem Rühren bei Raumtemperatur werden unter Kühlung 1,09 g (10 mMol) Propoxymethylchlorid zugegeben und über Nacht gerührt. Das Reaktionsgemisch wird im Vakuum eingeengt, der Rückstand mit Wasser versetzt und zweimal mit Methylenchlorid extrahiert. Die vereinigten organischen Phasen werden über Natriumsulfat getrocknet und eingeengt, der Rückstand in Diisopropylether aufgenommen, erneut eingeengt und chromatographisch an Kieselgel (Acetonitril/Wasser : 70/30) gereinigt.

Man erhält 0,51 g (29 % der Theorie) 3-(2-Chlor-thiazol-5-yl-methyl)-4-nitroimino-5-n-propoxymethyl-perhydro-1,3,5-oxadiazin mit einem logP = 1,70 (pH = 2).

[logP = Die Bestimmung der logP-Werte erfolgte gemäß EEC-Directive 79/831 Annex V. A8 durch HPLC (Gradientenmethode, Acetonitril/0,1 % wässrige Phosphorsäure]

Analog Herstellungsbeispiel 1 und gemäß den allgemeinen Angaben zur Herstellung der Verbindungen der Formel (I) werden die in der folgenden Tabelle 1 aufgeführten Verbindungen erhalten:

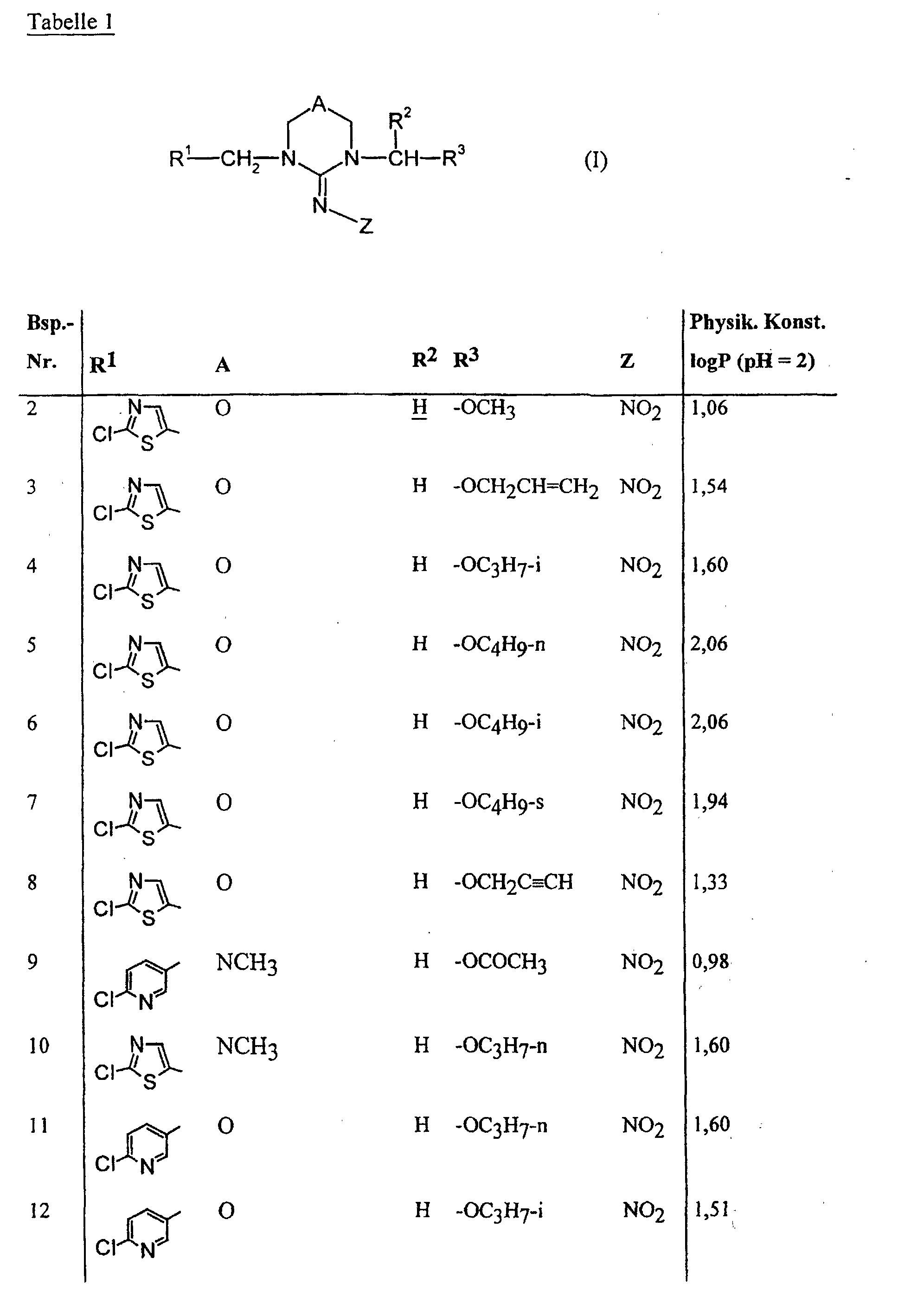

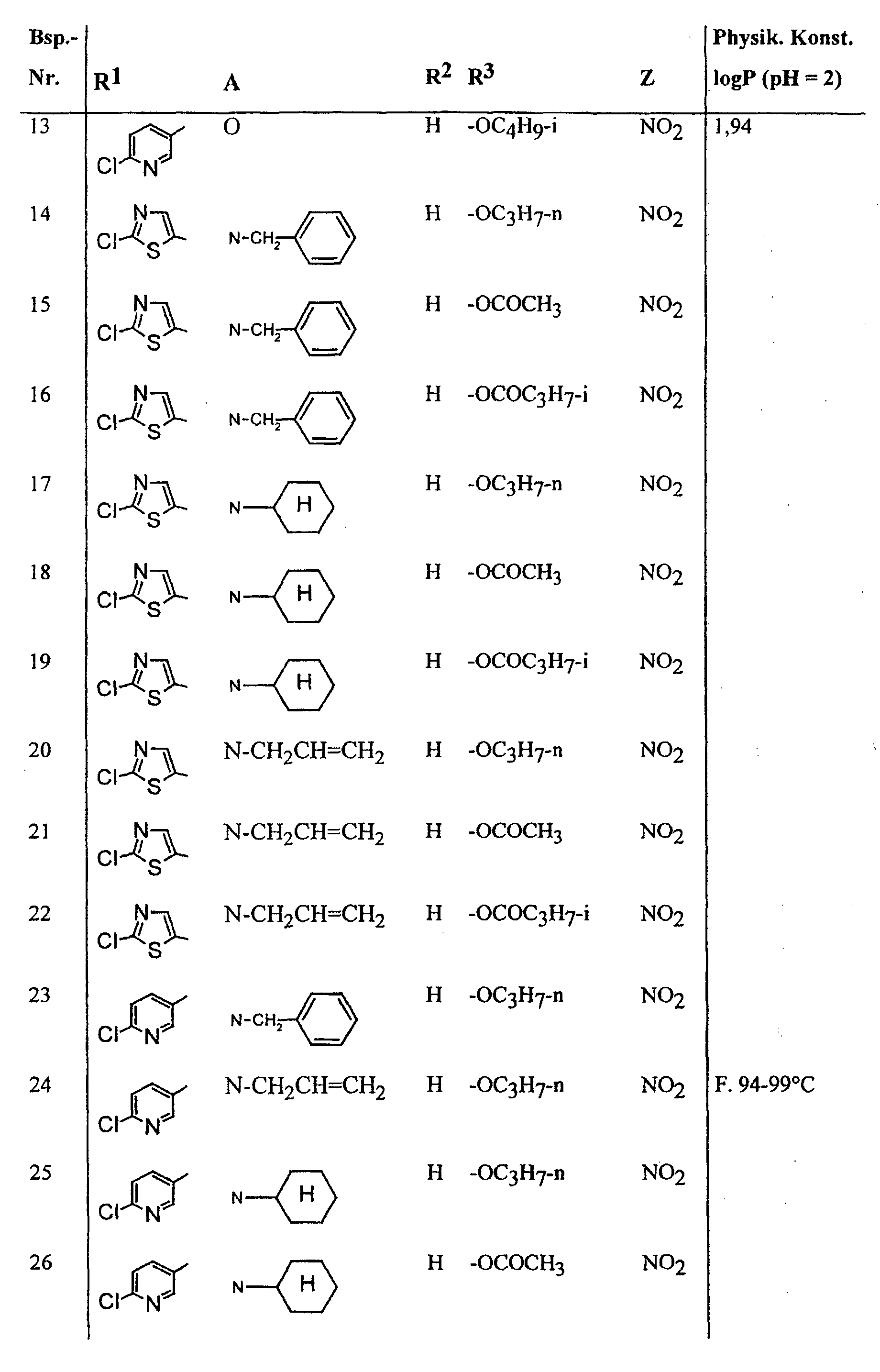

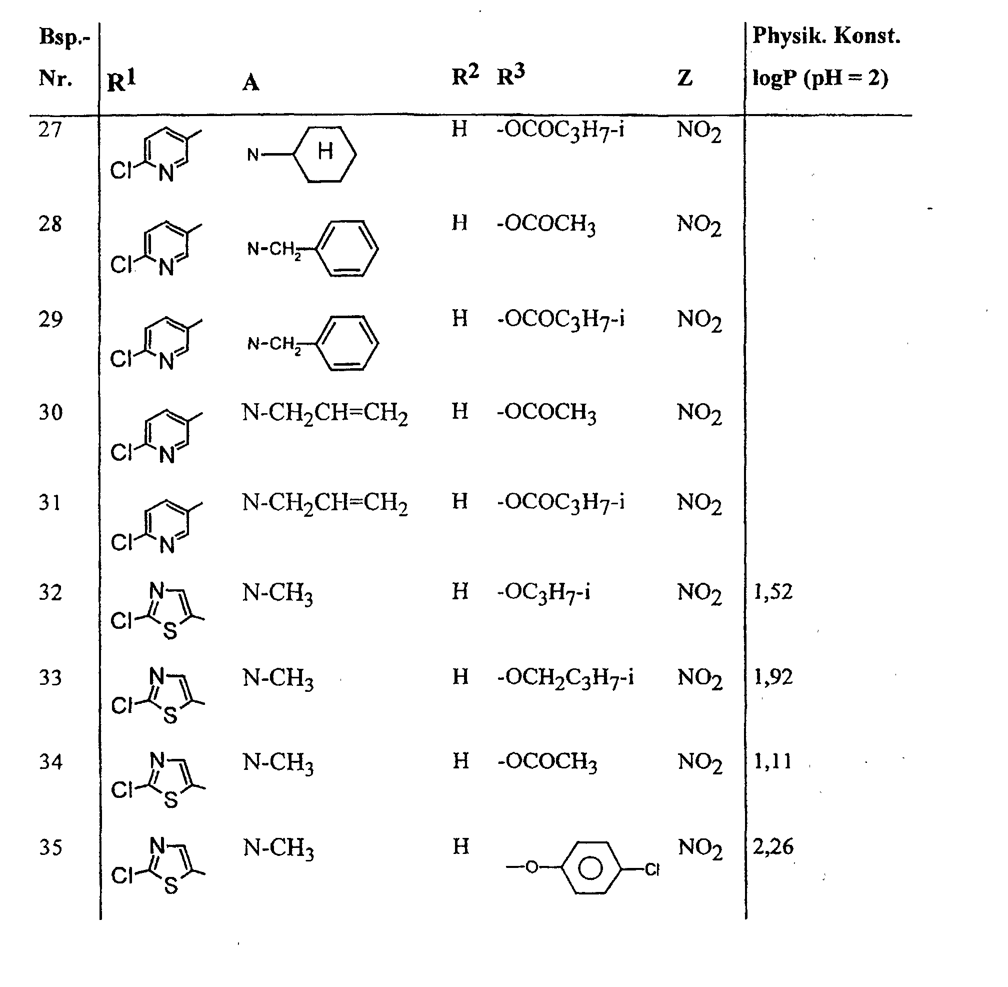

### Anwendungsbeispiele

### Beispiel A

### Myzus-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

In diesem Test bewirkten bei einer beispielhaften Wirkstoffkonzentration von 0,1 % z. B. die Verbindungen gemäß den Herstellungsbeispielen 1, 2, 3, 4, 5, 6, 7 und 8 eine Abtötung von 100 %, jeweils nach 6 Tagen.

### Beispiel B

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, dass alle Käfer-Larven abgetötet wurden; 0 % bedeutet, dass keine Käfer-Larven abgetötet wurden.

In diesem Test bewirkten bei einer beispielhaften Wirkstoffkonzentration von 0,1 % z. B. die Verbindungen gemäß den Herstellungsbeispielen 1, 2, 3, 4, 5, 6, 7 und 8 eine Abtötung von 100 %, jeweils nach 7 Tagen.

### Beispiel C

### Blowfly-Larven-Test/Entwicklungshemmende Wirkung

| | |
|---|---|
| Testtiere | Lucilia cuprina-Larven |
| Lösungsmittel | Dimethylsulfoxid |

20 mg Wirkstoff werden in 1 ml Dimethylsulfoxid gelöst, geringere Konzentrationen werden durch verdünnen mit Dest H20 hergestellt.

Etwa 20 Lucilia cuprina-Larven werden in ein Teströhrchen gebracht, welches ca. 1 cm³ Pferdefleisch und 0,5 ml der zu testenden Wirkstoffzubereitung enthält. Nach 24 Stunden und 48 Stunden wird die Wirksamkeit der Wirkstoffzubereitung ermittelt. Die Teströhrchen werden in Becher mit Sand-bedecktem Boden überführt. Nach weiteren 2 Tagen werden die Teströhrchen entfernt und die Puppen ausgezählt.

Die Wirkung der Wirkstoffzubereitung wird nach der Zahl der geschlüpften Fliegen nach 1,5-facher Entwicklungsdauer einer unbehandelten Kontrolle beurteilt. Dabei bedeutet 100 %, dass keine Fliegen geschlüpft sind; 0 % bedeutet, dass alle Fliegen normal geschlüpft sind.

In diesem Test zeigten bei einer beispielhaften Wirkstoffkonzentration von 100 ppm z. B. die Verbindungen gemäß den Herstellungsbeispielen 3 und 4 eine entwicklungshemmende Wirkung von 100 %.

### Beispiel D

### Test mit Fliegen (Musca domestica

| | |
|---|---|
| Testtiere | adulte Musca domestica, Stamm Reichswald (OP, SP, Carbamat-resistent) |
| Lösungsmittel | Dimethylsulfoxid |

20 mg Wirkstoff werden in 1 ml Dimethylsulfoxid gelöst, geringere Konzentrationen werden durch verdünnen mit Dest H₂O hergestellt.

2 ml dieser Wirkstoffzubereitung werden auf Filterpapierschalen (φ 9,5 cm) pipettiert, die sich in Petrischalen entsprechender Größe befinden. Nach Trocknung der Filterscheiben werden 25 Testtiere in die Petrischalen überführt und abgedeckt.

Nach 1, 3, 5 und 24 Stunden wird die Wirksamkeit der Wirkstoffzubereitung ermittelt. Dabei bedeutet 100 %, dass alle Fliegen abgetötet wurden; 0 % bedeutet, dass keine Fliegen abgetötet wurden.

In diesem Test bewirkte bei einer beispielhaften Wirkstoffkonzentration von 100 ppm z. B. die Verbindung gemäß dem Herstellungsbeispiel 8 eine Abtötung von 100%.

## Patentansprüche

1. Verbindungen der Formel (I) 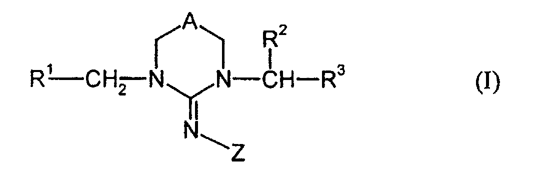 in welcher
R¹ für einen fünf- oder sechsgliedrigen Heterocyclus steht, welche 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt - und welcher gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenälkenyloxy, Alkinyloxy, Alkylthio, Halogenalkylthio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Arylthio, Arylamino, Aralkyl, Formylamino, Alkylcarbonylamino, Formyl, Carbamoyl, Alkylcarbonyl und/oder Alkoxycarbonyl substituiert ist,
R² für Wasserstoff oder Alkyl steht,
R³ für einen Rest aus der Reihe -OR⁴, -OCOR⁵, -OCOOR⁶, -OCONR⁷R⁸ und -OSO₂R⁹ und -S(O)ₙR¹⁰ steht, wobei
R⁴_{,} R⁵, R⁶ und R¹⁰ unabhängig voneinander für einen Rest aus der Reihe Alkyl, Alkoxyalkyl, Halogenalkyl, Alkenyl, Alkinyl, Alkylaminoalkyl, Dialkylaminoalkyl, gegebenenfalls substituiertes Cycloalkyl und jeweils gegebenenfalls substituiertes Phenyl und Benzyl stehen,
n für 0, 1 oder 2 steht,
R⁷ und R⁸ unabhängig voneinander für einen Rest aus der Reihe Wasserstoff, Alkyl, Alkenyl und jeweils gegebenenfalls substituiertes Phenyl und Benzyl stehen und
R⁹ für Alkyl oder gegebenenfalls substituiertes Phenyl steht,
A für Sauerstoff, Schwefel oder die Gruppierung -NR¹¹ steht, wobei
R¹¹ für einen Rest aus der Reihe Alkyl, Alkenyl, Alkinyl, Alkoxy, gegebenenfalls substituiertes Cycloalkyl oder gegebenenfalls substituiertes Aralkyl steht und
Z für Cyano oder Nitro steht.

2. Verfahren zur Herstellung von Verbindungen der Formel (I) gemäß Anspruch 1, **dadurch gekennzeichnet, dass** man Verbindungen der Formel (II) 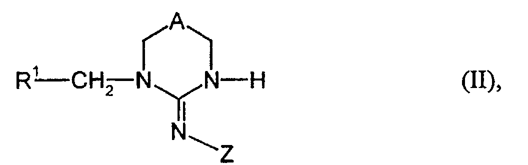 in welcher
R¹, A und Z die oben angegebenen Bedeutungen haben,
mit Verbindungen der Formel (III) 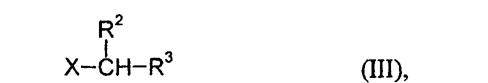 in welcher
R² und R³ die oben angegebenen Bedeutungen haben und
X für Halogen steht,
in Gegenwart einer Base und gegebenenfalls in Gegenwart eines Verdünnungsmittels umsetzt.

3. Schädlingsbekämpfungsmittel, **gekennzeichnet durch** einen Gehalt an mindestens einer Verbindung der Formel (I) gemäß Anspruch 1.

4. Verwendung von Verbindungen der Formel (I) gemäß Anspruch 1 zur Bekämpfüng von Schädlingen wobei die therapeutische Behandlung des menschlichen oder tierischen körpers ausgeschlossen ist.

5. Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt wobei die therapeutische Behandlung des menschlichen oder tierischen körpers ausgeschlossen ist.

6. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Mitteln vermischt.

7. Verwendung von Verbindungen der Formel (I) gemäß Anspruch 1 zur Herstellung von Schädlingsbekämpfungsmitteln.

## Claims

1. Compounds of the formula (I) 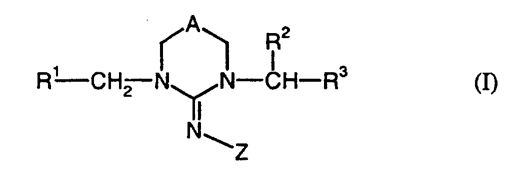 in which
R¹ represents a five- or six-membered heterocycle which contains 1, 2, 3 or 4 nitrogen atoms and/or one or two oxygen or sulfur atoms as heteroatom ring members - where the number of heteroatoms is 1, 2, 3 or 4 - and which is optionally substituted by halogen, cyano, nitro, alkyl, halogenoalkyl, alkenyl, halogenoalkenyl, alkinyl, alkoxy, halogenoalkoxy, alkenyloxy, halogenoalkenyloxy, alkinyloxy, alkylthio, halogenoalkylthio, alkenylthio, halogenoalkenylthio, alkinylthio, alkylsulfinyl, halogenoalkylsulfinyl, alkylsulfonyl, halogenoalkylsulfonyl, amino, alkylamino, dialkylamino, aryl, arylthio, arylamino, aralkyl, formylamino, alkylcarbonylamino, formyl, carbamoyl, alkylcarbonyl and/or alkoxycarbonyl,
R² represents hydrogen or alkyl,
R³ represents a radical from the group consisting of -OR⁴, -OCOR⁵, -OCOOR⁶, -OCONR⁷R⁸ and -OSO₂R⁹ and -S(O)ₙR¹⁰, where
R⁴, R⁵, R⁶ and R¹⁰ independently of one another represent a radical from the group consisting of alkyl, alkoxyalkyl, halogenoalkyl, alkenyl, alkinyl, alkylaminoalkyl, dialkylaminoalkyl, optionally substituted cycloalkyl and in each case optionally substituted phenyl and benzyl,
n represents 0, 1 or 2,
R⁷ and R⁸ independently of one another represent a radical from the group consisting of hydrogen, alkyl, alkenyl and in each case optionally substituted phenyl and benzyl and
R⁹ represents alkyl or optionally substituted phenyl,
A represents oxygen, sulfur or the group -NR¹¹, where
R¹¹ represents a radical from the group consisting of alkyl, alkenyl, alkinyl, alkoxy, optionally substituted aralkyl and
Z represents cyano or nitro.

2. Process for preparing compounds of the formula (I) according to Claim 1, **characterized in that** compounds of the formula (II) 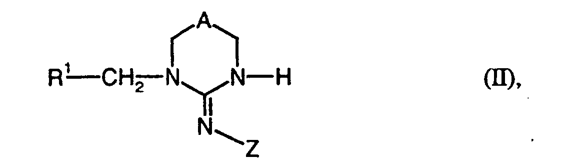 in which
R¹_{,} A and Z are as defined above
are reacted with compounds of the formula (III) 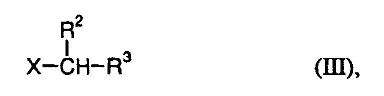 in which
R² and R³ are as defined above and
X represents halogen,
in the presence of a base and, if appropriate, in the presence of a diluent.

3. Pesticides, **characterized in that** they comprise at least one compound of the formula (I) according to Claim 1.

4. Use of compounds of the formula (I) according to Claim 1 for controlling pests, the therapeutic treatment of the human or animal body being excluded.

5. Method for controlling pests, **characterized in that** compounds of the formula (I) according to Claim 1 are allowed to act on pests and/or their habitat, the therapeutic treatment of the human or animal body being excluded.

6. Process for preparing pesticides, **characterized in that** compounds of the formula (I) according to Claim 1 are mixed with extenders and/or surfactants.

7. Use of compounds of the formula (I) according to Claim 1 for preparing pesticides.

## Revendications

1. Composés de la formule (I) : 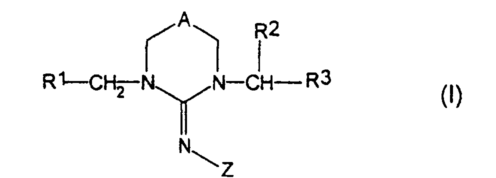 dans laquelle :
R¹ représente un hétérocycle à cinq ou six membres, qui contient 1, 2, 3 ou 4 atomes d'azote et/ou un ou deux atomes d'oxygène ou de soufre comme membre du cycle hétéroatomique, où le nombre d'hétéroatomes s'élève à 1, 2, 3 ou 4, et qui est le cas échéant substitué par un atome d'halogène, le radical cyano, nitro, un radical alcoyle, halogénoalcoyle, alcényle, halogénoalcényle, alcynyle, alcoxy, halogénoalcoxy, alcényloxy, halogénoalcényloxy, alcynyloxy, alcoylthio, halogénoalcoylthio, alcénylthio, halogénoalcénylthio, alcynylthio, alcoylsulfinyle, halogénoalcoylsulfinyle, alcoylsulfonyle, halogénoalcoylsulfonyle, amino, alcoylamino, dialcoylamino, aryle, arylthio, arylamino, aralcoyle, formylamino, alcoylcarbonylamino, formyle, carbamoyle, alcoylcarbonyle et/ou alcoxycarbonyle ;
R² représente l'atome d' hydrogène ou un radical alcoyle ;
R³ représente un reste de la série -OR⁴, -OCOR⁵, -OCOOR⁶, -OCONR⁷R⁸, -OSO₂R⁹ et -S(O)ₙR¹⁰, où
_{R}⁴, _{R}⁵, R⁶ et R¹⁰ représentent indépendamment l'un de l'autre, un reste de la série des radicaux alcoyle, alcoxyalcoyle, halogénoalcoyle, alcényle, alcynyle, alcoylaminoalcoyle, dialcoylaminoalcoyie, cycloalcoyle le cas échéant substitué et phényle et benzyle, chaque fois le cas échéant substitués ;
n représente 0, 1 ou 2 ;
R⁷ et R⁸ représentent indépendamment l'un de l'autre, un reste de la série de l'atome d'hydrogène, des radicaux alcoyle, alcényle et phényle et benzyle, chaque fois, le cas échéant substitués, et
R⁹ représente un radical alcoyle ou le radical phényle le cas échéant substitué ;
A représente l'atome d'oxygène, de soufre ou le radical NR¹¹, où
R¹¹ représente un reste de la série des radicaux alcoyle, alcényle, alcynyle, alcoxy, cycloalcoyle le cas échéant substitué et aralcoyle le cas échéant substitué ;
Z représente le radical cyano ou nitro.

2. Procédé de préparation des composés de la formulé (I) suivant la revendication 1, **caractérisé en ce que** l'on fait réagir les composés de la formule (II) : 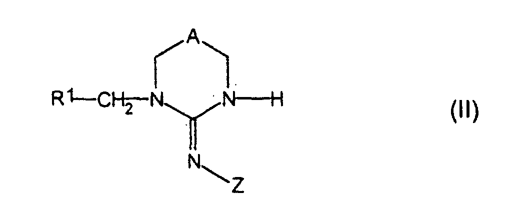 dans laquelle :
R¹, A et Z ont les significations indiquées ci-dessus,
avec les composés de la formule (III) : 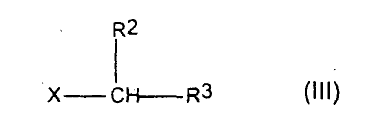 dans laquelle :
R² et R³ ont les significations indiquées ci-dessus, et
X représente un atome d'halogène,
en présence d'une base et le cas échéant, en présence d'un agent de dilution.

3. Agent de lutte contre les parasites, **caractérisé par** une teneur en au moins un composé de la : formule (I) suivant la revendication 1.

4. Utilisation des composés de la formule (I) suivant la revendication 1, pour lutter contre la parasites, où le traitement thérapeutique . du corps humain ou animal est exclu.

5. Procédé pour lutter contre les parasites, **caractérisé en ce que** l'on fait agir les composés de la formule (I) suivant la. revendication 1, sur les parasites et/ou leur biotope, où le traitement thérapeutique du corps humain ou animal est exclu.

6. Procédé pour préparer un agent de lutte contre les parasites, **caractérisé en ce que** l'on mélange les composés de la formule (I) suivant la revendication 1 1 avec un agent de dilution et/ou des agents tensioactifs.

7. Utilisation des composés de la formule (I) suivant la revendication 1, pour la préparation d'agents de lutte contre les parasites.
